# EUROPEAN PATENT APPLICATION

(11) **EP 2 202 313 A1**
(43) Date of publication of application: **30.06.2010**
(21) Application number: 08792415.5
(22) Date of filing: 12.08.2008
(51) Int. Cl.: C12N 15/66

(54) **METHOD FOR PREPARATION OF RECOMBINANT DNA**

(30) Priority: 21.08.2007 JP 2007215238
(71) Applicant: National University Corporation Hokkaido University, Sapporo-shi Hokkaido 060-0808 (JP)
(72) Inventor: NAGAI, Takeharu, Sapporo-shi Hokkaido 001-0021 (JP); KOTERA, Ippei, Sapporo-shi Hokkaido 001-0021 (JP)
(74) Representative: Niemann, Frédéric
(86) International application number: PCT/JP2008/064482
(87) International publication number: WO 2009/025209

(57) **Abstract**

The problem to be solved in the present invention is to provide a simplified and efficiently improved DNA recombination method.

The above problem can be solved with the present method for preparing a recombinant DNA by inserting a DNA fragment of interest into a vector DNA, the method comprising the step of carrying out the following reactions at the same reacting location at the substantially simultaneouse time: (a) a reaction for simultaneously cleaving a site of the vector for inserting the fragment and a DNA containing the fragment in the presence of a restriction enzyme whose DNA recognition site and DNA cleavage site are discrete; and (b) a reaction for inserting the fragment into the vector in the presence of a DNA ligase.

## Description

### FIELD OF THE INVENTION

The present invention relates to a method for preparing a recombinant DNA, more particularly, to a method for preparing a recombinant DNA wherein a DNA fragment of interest is efficiently inserted into a vector DNA by simplified steps.

### BACKGROUND OF THE INVENTION

Genetic recombination techniques have been put into practical since the 1970s, and currently similar methods are still in use extensively. Specifically, according to this conventional method, a vector (mediator) of a gene called plasmid is cleaved with a restriction enzyme(s), to which a gene of interest is ligated by a DNA ligase. This method has allowed extensive genetic recombination, and has significantly affected various fields.

For recombination of a gene of interest, first, the gene of interest and a vector need to be mass-prepared by using a PCR method, *E*. *coli*, lambda phage or the like. Then, both ends of the gene of interest and the vector are processed with a restriction enzyme(s) so that each end has a shape that allows complementary hybridization. In order to prevent self-ligation of the vector, the vector DNA may be dephosphorylated. Subsequently, only DNA fragments having the size of interest are separated and purified by electrophoresis or the like. The resulting DNA fragments and the vectors are mixed together in a test tube at a certain molar ratio, which are processed by a DNA ligase to obtain a circular DNA in which the vector and the DNA fragment of interest are linked to each other. This circular DNA is used to transform a host cell such as *E*. *coli,* and then the resulting *E*. *coli* colonies are screened for specifying the *E*. *coli* bearing the gene of interest. The resulting recombinant *E*. *coli* is cultured, from which plasmid DNA is purified, thereby obtaining a plasmid carrying the gene of interest.

The above-described conventional method, however, requires complicated and cumbersome manipulations, which require substantial time to become skilled. In addition, since most part of the conventional method is carried out manually, researchers have to spare most of their time for the operation of genetic recombination in studies requiring genetic recombination.

Recently, a method was reported for preparing a vector for directional cloning in which the direction of a nucleotide fragment produced with a restriction enzyme is controlled (for example, see Japanese Laid-Open Patent Application No. 2007-508012).
Patent Reference 1: Japanese Laid-Open Patent Application No. 2007-508012

### DISCLOSURE OF THE INVENTION

### Problems to be Solved by the Invention

In view of the above-described problems in conventional techniques, there has been a need for the development of a simplified and efficiently improved DNA recombination method.

### Means for Solving the Problems

In order to solve the above-mentioned problems, the present inventors have gone through keen research and found that combinational use of certain restriction enzyme with certain DNA ligase can simplify a multistep process to perform convenient and efficient DNA recombination, thereby accomplishing the present invention. Thus, the present invention provides a method for preparing a recombinant DNA as described below.
(1)
   (i) A method for preparing a recombinant DNA by inserting a DNA fragment of interest into a vector DNA, comprising the step of carrying out the following reactions at the same reacting location at the substantially simultaneous time:
      (a) a reaction for simultaneously cleaving a site of the vector DNA for inserting the fragment and a DNA containing the fragment in the presence of a restriction enzyme whose DNA recognition site and DNA cleavage site are discrete; and
      (b) a reaction for inserting the fragment into the vector DNA in the presence of a DNA ligase.
   (ii) A method for preparing a recombinant DNA by inserting a DNA fragment of interest into a vector DNA, comprising the step of carrying out the following reactions at the same reacting location at the substantially simultaneous time:
      (a) a reaction for simultaneously cleaving a site of the vector DNA for inserting the fragment and both ends of a DNA containing the fragment in the presence of a restriction enzyme whose DNA recognition site and DNA cleavage site are discrete; and
      (b) a reaction for inserting the fragment into the vector DNA in the presence of a DNA ligase.
(2) The method according to (1) above, wherein the restriction enzyme recognizes and cleaves, as a DNA cleavage site, a site at a certain distance from the DNA recognition site.
(3) The method according to (1) above, wherein the restriction enzyme is a Class-IIS (also called Type-IIS) restriction enzyme.
(4) The method according to any one of (1) to (3) above, wherein the DNA fragment of interest consists of multiple DNA fragments each having a different sequence, and wherein the multiple DNA fragments are simultaneously inserted into the vector DNA.
(5) The method according to any one of (1) to (4) above, wherein the reactions (a) and (b) are carried out by using a solution obtained by adding a DNA polymerase inhibitor to a PCR solution containing a PCR-amplified DNA including the DNA fragment of interest and a PCR-amplified vector DNA.
   The present invention further provides the following methods.
(6) The method according to any one of (1) to (5) above, wherein the restriction enzyme recognizes a DNA recognition site having four or more bases.
(7) The method according to (6) above, wherein the restriction enzyme recognizes a DNA recognition site having seven or more bases.
(8) The method according to any one of (3) to (5) above, wherein the Class-IIS restriction enzyme is *Lgu*I, *Bfu*AI, *Fok*I, *Bsm*AI, *Bsm*FI, *Sfa*NI or *Bbv*I.
(9) The method according to (8) above, wherein the Class-IIS restriction enzyme is *Lgu*I.
(10) The method according to (8) above, wherein the Class-IIS restriction enzyme is *Bfu*AI.
(11) The method according to any one of (1) to (10) above, wherein the DNA ligase is T4 DNA ligase, *E*. *coli* DNA ligase or T7 DNA ligase.
(12) The method according to any one of (1) to (11) above, wherein the vector DNA is a plasmid vector DNA.
(13) The method according to any one of (1) to (12) above, wherein the vector DNA, the DNA containing the fragment of interest, the restriction enzyme and the DNA ligase are simultaneously brought into contact with one another in the same reaction vessel.
(14) The method according to any one of (1) to (13) above, wherein the DNA fragment of interest comprises a gene.
(15) The method according to any one of (5) to (14) above, wherein the polymerase inhibitor is aphidicolin.

Moreover, the present invention also provides the following methods.
(16) The method according to any one of (1) to (15) above, wherein the vector DNA and the DNA containing the fragment of interest comprise the DNA recognition site of the restriction enzyme.
(17) The method according to any one of (1) to (16) above, wherein the vector DNA and the DNA containing the fragment of interest comprise the DNA cleavage sites for the restriction enzyme that are complementary to each other.

### EFFECT OF THE INVENTION

According to a DNA recombination method of the present invention described as above, the operation time and labor for DNA recombination can be greatly reduced. According to the present invention, DNA recombination can be carried out efficiently. In a preferred embodiment of the present invention, simultaneous insertion of multiple DNA fragments into a vector can readily be realized which has been very difficult with conventional methods.

### BRIEF DESCRIPTION OF THE DRAWING

Figure 1 is a view illustrating a method for preparing a recombinant DNA by incorporating a DNA fragment of interest into a vector DNA according to the present invention.
Figure 2 shows a schematic view and primer sequences for DNA recombination using *Lgu*I in Example 1.
Figure 3 shows the results of electrophoresis of PCR products obtained in Example 1.
Figure 4 shows the results of colony direct PCR obtained in Example 1.
Figure 5 shows the results of nucleotide sequence analysis in the vicinity of the ligation site by DNA sequencing in Example 1.
Figure 6 shows a schematic view and primer sequences for DNA recombination using *Bfu*AI in Example 2.
Figure 7 shows the results of colony direct PCR obtained in Example 2.
Figure 8 shows the results of expression efficiency of the recombinant vector DNA obtained in Example 3 in *E.coli.*

### BEST MODES FOR CARRYING OUT THE INVENTION

Hereinafter, the present invention will be described in detail.

The term "recombination" as used in the present invention refers to a technique in which a certain foreign DNA is introduced into a particular individual to modify the genetic information of the individual. The term "recombinant DNA" as used herein refers to a hybrid DNA molecule containing at least two nucleotide sequences that cannot usually be found together in nature.

A first embodiment of the present invention is a method for preparing a recombinant DNA by inserting a DNA fragment of interest into a vector DNA, comprising the step of carrying out the following reactions (a) and (b) at the same reacting location at the simultaneous time at the simultaneous time.
(a) A reaction for simultaneously cleaving a site of the vector DNA for inserting the fragment and a DNA containing the fragment in the presence of a restriction enzyme whose DNA recognition site and DNA cleavage site are discrete.
(b) A reaction for inserting the fragment into the vector DNA in the presence of a DNA ligase.

Reaction (a) is for simultaneously cleaving a site of vector DNA for inserting the DNA fragment of interest and a DNA containing the fragment in the presence of a restriction enzyme whose DNA recognition site and DNA cleavage site are discrete. The DNA containing the fragment may be cleaved at both ends in reaction (a).

Herein, the term "restriction enzyme" refers to an enzyme that recognizes and cleaves a specific nucleotide sequence of DNA.

A restriction enzyme whose DNA recognition site and DNA cleavage site are discrete as used herein refers to a restriction enzyme that binds to but does not cleave a DNA at the recognition site or a specific double-stranded DNA sequence called a recognition nucleotide sequence, and recognizes, as the DNA cleavage site, a sequence at a site at least 1 bp away (preferably, a site at a predetermined distance) from its binding site, thereby cleaving the double-stranded DNA. Although the type of the restriction enzyme used in the present invention is not particularly limited as long as it has the above-described property, it is preferably a type II restriction enzyme, and particularly preferably a type II restriction enzyme whose DNA recognition site is of four or more bases, five or more bases, six or more bases, particularly seven or more bases, and preferably 20 or less bases, 18 or less bases, 16 or less bases, 14 or less bases, or 12 or less bases. The cleaving manner of a type II restriction enzyme may be one that results either a blunt end or a sticky end, but it is preferably one that causes a sticky end. A restriction enzyme used in the present invention is characterized by having a function of specifically cleaving a site distant from the DNA recognition site, where the cleaved fragment will have a sticky end that consists of several (2 to 6) bases of a variable sequence. For example, a Class-IIS restriction enzyme can preferably be used. A Class-IIS restriction enzyme is known to cleave double-stranded DNA at a cleavage site that is a predetermined distance (up to 20 bp) away from the asymmetric recognition site of the enzyme (Szynbalski, W., Gene, 40:169, 1985).

Specific examples of "Class-IIS restriction enzymes" used in the present invention include but not limited to the followings. In the following examples, the Class-IIS restriction enzymes are classified according to the protrusion types of the sticky ends of the polynucleotide fragments produced through their cleavage.

### (Examples of Class-IIS restriction enzymes)

One-base-protrusion sticky end type:
*Asu*HPI, *Bci*VI, *Bfi*I, *Bfu*I, *Bmr*I, *Bmu*I, *Hin4*II, *Hph*I, *HpyAV*, *Mbo*II, *Mnl*I, *Ncu*I, *Acl*WI, *Alw*I, *Bcc*I, *Bcef*I, *Bin*I, *Bsp*PI, *Bst*H91, *Bst*31TI, *Eac*I, *Hpy*C1I, *Pde*I, *Pps*I

Two-base-protrusion sticky end type:
*Acu*I, *Asp*26HI, *Asp*27HI, *Asp*35HI, *Asp*36HI, *Asp*40HI, *Asp*50HI, *Bce*83I, *Bcg*I, *Bcg*I, *Bma*HI, *Bpm*I, *Bpu*EI, *Bsa*MI, *Bsc*CI, *Bse*1I, *Bse3*DI, *Bse*GI, *Bse*MI, *Bse*MII, *Bse*NI, *Bse*RI, *Bsg*I, *Bsm*I, *Bsp*CNI, *Bsp*KT5I, *Bsr*I, *Bsr*DI, *Bsr*SI, *Bst*11I, *Bst*F5I, *Bts*I, *Bts*CI, *Csp*CI, *Csp*CI, *Cst*MI, *Eci*I, *Eco*57I, *Eco*57MI, *Gsu*I, *Mme*I, *Mva*1269I, *Nme*AIII, *Pct*I, *Taq*II, *Taq*II, *Tso*I, *Tsp*1I, *Tsp*DTI, *Tsp*GWI, *Tth*111II, *Aci*I, *Bce*AI, *Bme*585I, *Bsc*AI, *Bsp*ACI, *Bst*19I, *Bst*FZ438I, *Fau*I, *Smu*I, *Ssi*I

Three-base-protrusion sticky end type:
*Bsa*XI, *Bsa*XI, *Rle*AI, *Abe*I, *Bbv*CI, *Bco*5I, *Bco*116I, *Bco*KI, *Bpu*10I, *Bpu*DI, *Bse*ZI, *Bsp*QI, *Bss*IMI, *Bst*6I, *Bsu*6I, *Eam*1104I, *Earl*, *Ksp*632I, *Lgu*I, *Pci*SI, *Sap*I, *Sim*I, *Vpa*K32I

Four-base-protrusion sticky end type:
*Aar*I, *Acc36*I, *Ace*III, *Alw*26I, *Adw*XI, *Bau*I, *Bbr7*I, *Bbs*I, *Bbv*I, *Bbv*II, *Bbv*16II, *Bfu*AI, *Bli*736I, *Bpi*I, *Bpu*AI, *Bpu*SI, *Bsa*I, *Bsc*91I, *Bse*KI, *Bse*XI, *Bse*YI, *Bsi*I, *Bsl*FI, *Bsm*AI, *Bsm*BI, *Bsm*FI, *Bso*31I, *Bso*MAI, *Bsp*423I, *Bsp*BS31I, *Bsp*IS4I, *Bsp*LU11III, *Bsp*MI, *Bsp*ST5I, *Bsp*TNI, *Bsp*TS514I, *Bss*SI, *Bst*12I, *Bst*71I, *Bst2B*I, *Bst*BS32I, *Bst*GZ53I, *Bst*MAI, *Bst*OZ616I, *Bst*TS5I, *Bst*V1I, *Bst*V2I, *Btg*ZI, *Bve*I, *Eco*31I, *Eco*A4I, *Eco*O44I, *Esp3*I, *Faq*I, *Fok*I, *Gdi*II, *Lwe*I, *Pha*I, *Sfa*NI, *Sth132*I, *Sts*I

Five-base-protrusion sticky end type:
*Aju*I, *Aju*I, *Alo*I, *Ado*I, *Bae*I, *Bae*I, *Bsp*24I, *Bsp*24I, *Hin4*I, *Hin4*I, *Ppi*I, *Ppi*I, *Psr*I, *Psr*I, *Tst*I, *Tst*I, *Cse*I, *Hga*I

Six-base-protrusion sticky end type:
*Cje*I, *Cje*I, *CjeP*I, *CjeP*I

In addition, isoschizomers of the above enzymes may also be used as Class-IIS restriction enzymes.

Above all, *Lgu*I (from Cosmo Bio Co., Ltd. (Fermentas)), *Bfu*AI, *Fok*I (from Takara Shuzo Co., Ltd.), *Bsm*AI, *Bsm*FI (from Biolab), *Sfa*NI or *Bbv*I is preferably used.

The reaction step (a) may be carried out under conditions known by those skilled in the art (Reference: Gene. 1985; 40 (2-3):169-73. Universal restriction endonucleases: designing novel cleavage specificities by combining adapter oligodeoxynucleotide and enzyme moieties.). A reaction solution used here is a buffer suitable for reaction with the restriction enzyme. For example, Tris-HCl, Tris-acetate, K-acetate, MgCl₂, Mg-acetate, DTT, 2-mercaptoethanol, BSA, ATP or the like may preferably be combined. The temperature of this reaction system is, for example, within a range of 15 to 90°C, preferably within a range of 25 to 50°C.

The term "a DNA fragment of interest" as used herein generally refers to a DNA fragment as a target to be inserted into a vector to express a protein coded by the DNA fragment. Although the DNA fragment of interest is preferably a DNA containing a gene, it may also be the DNA fragment containing other than a gene such as a promoter, an intron, an RNAi vector or the like. The DNA fragment of interest may further include a sticky end sequence, a linker sequence required for insertion into a vector or for preparation of a reading frame of the gene, or a sequence required for appropriate expression of the gene (e.g., Kozak sequence, SD sequence or terminator).

A DNA fragment of interest is produced by cleaving a DNA containing the fragment with a restriction enzyme in reaction (a).

Nucleic acids (DNA) containing the above-mentioned DNA fragment of interest such as a gene or the like may readily be prepared by those skilled in the art, by utilizing general genetic recombination techniques (for example, see Molecular Cloning: A laboratory Manual, 3rd ed., Cold Spring Harbor Laboratory, Cold Spring Harbor, NY., 2001). For example, PCR, RT-PCR or the like may be performed for amplification with primers designed to include the nucleic acids to be amplified. As a template, an RNA or a cDNA derived from a cell expressing such a gene, or a recombinant vector containing the DNA fragment of interest may be used. The DNA containing the fragment of interest may also be produced with *E*. *coli,* lambda phage or the like.

Furthermore, for reaction (a) of the present invention, a recombinant vector containing a DNA fragment of interest may be used as the DNA containing the fragment in the form where the fragment is contained in the vector .

In order to rapid DNA recombination according to the present invention, the vector should preferably be a PCR-amplified vector, namely a PCR product. When thePCR-amplified vector is used as a DNA containing a fragment of interest in reaction (a) of the present invention, the template DNA remaining in the PCR solution is preferably cleaved with *Dpn*I to be eliminated from the PCR solution in advance. *Dpn*I is a restriction enzyme that specifically cleaves methylated DNA.

Separation and purification of a DNA containing the fragment of interest may be carried out according to a known conventional method. For example, general separation/purification techniques such as electrophoresis using agarose gel may be employed.

If purification with removing contamination is necessary, a known conventional method may be performed. For example, a commercially available purification kit or the like may be used.

Although the length of the DNA containing the fragment of interest is not particularly limited, it is preferably 50 to 20000 bases, more preferably 100 to 10000 bases, and still more preferably 500 to 5000 bases (including the end moieties). The length of the sticky end moiety is also not particularly limited, but it is preferably 1 to 8 bases, preferably 2 to 4 bases.

The DNA fragment of interest used in the present invention may be a single gene, or multiple, meaning two or more species of, genes having different sequences. In terms of efficient recombination, the species of the DNA fragments of interest to be used are preferably four or less, providing that such multiple DNA fragments of interest can all be obtained by cleaving multiple of DNAs containing the fragments using the same restriction enzyme. The sticky ends at both ends of the DNA fragments of interest should be complementary such that they can be linked with each other. Furthermore, the sticky ends of the DNA fragments of interest that are linked with the vector DNA are preferably complementary with the sticky ends of the vector DNA that is simultaneously produced upon the reaction. In order to simultaneously insert multiple DNA fragments of interest into the vector DNA, multiple DNAs which contain the fragments to be used in reaction (a) should have the DNA cleavage sites of the restriction enzyme that are complementary to each other, while, among these DNAs, the DNA containing the fragment to be ligated with the vector DNA should have a DNA cleavage site complementary to the site of the vector DNA for inserting the fragment.

The vector DNA that can be used in the present invention may be any vector DNA, including, for example, a bacteriophage such as plasmid vector DNA, a phagemid vector, a lambda phage or the like, an animal virus such as retrovirus, vaccinia virus or the like, or nucleic acids capable of self-circularization such as a cosmid. For example, a plasmid vector such as an *E*. *codi*-derived plasmid (e.g., pBR325, pUC12, pUC13), a *Bacillus subtilis*-derived plasmid (e.g., pUB110, pTP5, pC194), or an yeast-derived plasmid (e.g., pSH19, pSH15) is preferably used.

Moreover, the vector DNA may be a commercially available vector, or it may readily be prepared by those skilled in the art by employing a general genetic recombination technique. Specifically, a method similar to the above-described method for preparing the DNA containing the fragment of interest, for example, amplification by a PCR method or the like, separation/purification by electrophoresis, or other methods may be used.

When a PCR-amplified vector DNA is used, the template DNA remaining in the PCR solution is preferably cleaved with *Dpn*I that specifically cleaves methylated DNA only to eliminate it from the PCR solution in advance. Treatment of the PCR reaction solution with *Dpn*I allows faster DNA recombination.

Although the length of the vector DNA is not particularly limited, it is preferably 500 to 20000 bases (including the sticky end moieties), more preferably 1000 to 10000 bases, and still more preferably 2000 to 5000 bases. The length of the sticky end moiety is also not particularly limited, but it is 1 to 8 bases, preferably 2 to 4 bases. The vector should be selected with appropriately considering the type of the restriction enzyme to be used, the transformation efficiency and else.

A site of vector DNA for inserting a DNA fragment of interest according to the present invention refers to a site of the vector DNA prepared for inserting the fragment. According to a preferred embodiment of the present invention, since the vector DNA and the DNA containing the fragment of interest used in reaction (a) have DNA cleavage sites complementary to each other, simultaneous cleaving the vector DNA and the DNA containing the fragment with a restriction enzyme such as a Class-IIS restriction enzyme that results sticky ends, can provide the processed vector DNA having both sticky ends complementary to the both ends of the DNA fragment.

Here, since a "DNA containing a DNA fragment of interest" and a "vector DNA" are simultaneously cleaved with a restriction enzyme in reaction (a) of the present invention, these DNAs need to have the DNA recognition sequence of the restriction enzyme used in reaction (a).

Furthermore, when a restriction enzyme that results sticky ends is used, the sticky ends of the vector DNA and the sticky ends of the DNA fragment of interest which are simultaneously prepared with the restriction enzyme in reaction (a) need to be complementary to each other so that the fragment and the vector DNA are ligated by a DNA ligase in reaction (b). To satisfy this, the DNA containing the fragment of interest and the vector DNA should contain the DNA cleavage sites of the restriction enzyme which are complementary to each other.

Specifically, the DNA containing the DNA fragment of interest needs to contain, in addition to the DNA recognition sequence of the restriction enzyme, a sticky end sequence complementary to a sticky end obtained by cleaving the site of the vector DNA for inserting the fragment with the same enzyme.

The vector DNA also needs to contain, in addition to the DNA recognition sequence of the restriction enzyme, a sticky end sequence complementary to a sticky end obtained by cleaving the DNA containing the DNA fragment of interest with the same enzyme.

Such DNA recognition sequence and sticky end sequence may be introduced into the DNA containing the DNA fragment of interest as well as the vector DNA by a known technique such as a PCR method. For example, primers designed to contain either or both of the DNA recognition sequence and the sticky end sequence can be used in the PCR method to amplify a DNA containing the fragment of interest or a vector DNA containing these sequences. Those skilled in the art would appropriately determine the base-numbers of the DNA recognition sequence and the sticky end sequence in the primers based on the property of the restriction enzyme to be used.

Alternatively, such DNA recognition sequence and sticky end sequence may be already contained in a DNA containing the DNA fragment of interest or the vector DNA, or they may be contained in a vector DNA employed for preparing these DNAs.

The DNA containing the DNA fragment of interest and the vector DNA, each having the DNA recognition sequence and the sticky end sequence prepared as described above, may be brought into contact with the restriction enzyme, thereby simultaneously providing the DNA fragment of interest and the vector DNA having sticky ends complementary to each other.

Next, reaction (b) is for inserting the DNA fragment of interest into the vector DNA in the presence of a DNA ligase to express the fragment. Reaction (b) may be carried out by bringing the DNA fragment of interest and the vector DNA obtained in reaction (a) into contact with a DNA ligase.

A DNA ligase used herein refers to an enzyme that ligates cleaved moieties of double-strand DNAs, by binding 5'-phosphate end and 3'-OH end of respective adjacent DNA strands via a phosphodiester bond. The DNA ligase as used herein may be any DNA ligase as long as the DNA fragment of interest and the site of the vector DNA for inserting the fragment that are obtained in reaction (a) can be ligated. Examples of the DNA ligase used in the present invention include T4 DNA ligase, T7 DNA ligase, *E*. *coli* DNA ligase, Ampligase (Epicentre Biotechnologies), *Pfu* ligase (Stratagene), *Rma* DNA ligase (Prokaria), *Tsc* DNA ligase (Prokaria), *Tth* DNA ligase (Eurogenetec), Taq ligase (NEB) and the like. Enzymes other than these exemplary enzymes may also be used in the present technique as long as it is an ATP- or NAD+-dependent, double-strand-specific ligase.

Among these, T4 DNA ligase, *E*. *coli* DNA ligase, T7 DNA ligase or the like may be preferably used.

According to the present invention, reactions (a) and (b) are carried out at the same reacting location at the substantially simultaneous time. For example, reactions (a) and (b) are simultaneously carried out in a single reaction vessel. The phrase "the substantially simultaneous time" means that the cleavage reaction of reaction (a) practically occurs immediately before the insertion reaction of reaction (b). Specifically, the vector DNA, the DNA containing the fragment of interest, the restriction enzyme and the DNA ligase are simultaneously brought into contact with one another in the same reaction vessel. More specifically, reactions (a) and (b) are carried out by mixing the four components of the DNA containing the fragment of interest, the vector DNA, the restriction enzyme and the DNA ligase, in an appropriate solution.

Alternatively, in order to insert DNA fragments of interest each having different gene sequences into a vector, two or more types of DNAs containing the fragments may be used and mixed at the same time.

The reaction solution used herein may be a buffer suitable for the restriction enzyme and the ligase. For example, Tris-HCl, Tris-acetate, K-acetate, MgCl₂, Mg-acetate, DTT, 2-mercaptoethanol, BSA, ATP, a commercially available premixed restriction enzyme/ligase buffer or the like may be used in a preferable combination. The temperature of the reaction system is, for example, within a range of 4 to 50°C, preferably 4 to 37°C. Specifically, reaction is preferably performed within an optimum reaction temperature range for the restriction enzyme and the ligase.

The reaction vessel is not particularly limited, but it may be heat- and acid-proof. A glass container such as Pyrex (registered trademark) or a test tube, or a plastic container such as polypropylene may preferably be used.

The concentrations of the four components of the DNA containing the DNA fragment of interest, the vector DNA, the restriction enzyme and the ligase upon mixing them in the solution are not particularly limited. For example, they are preferably mixed at around the following concentrations: the DNA containing the fragment of interest is 10 pM to 10 nM; the vector DNA is 10 pM to 10 nM; the restriction enzyme is 0.1 to 100 units/ml, preferably 0.1 to 10 units/ml; and the DNA ligase is 0.1 to 100 units/ml, preferably 0.1 to 10 units/ml.

Reaction time is not particularly limited but it is preferably 10 minutes to 3 hours, preferably 1 to 2 hours.

Hereinafter, a method for preparing a recombinant DNA according to the present invention will be described with reference to the drawings.

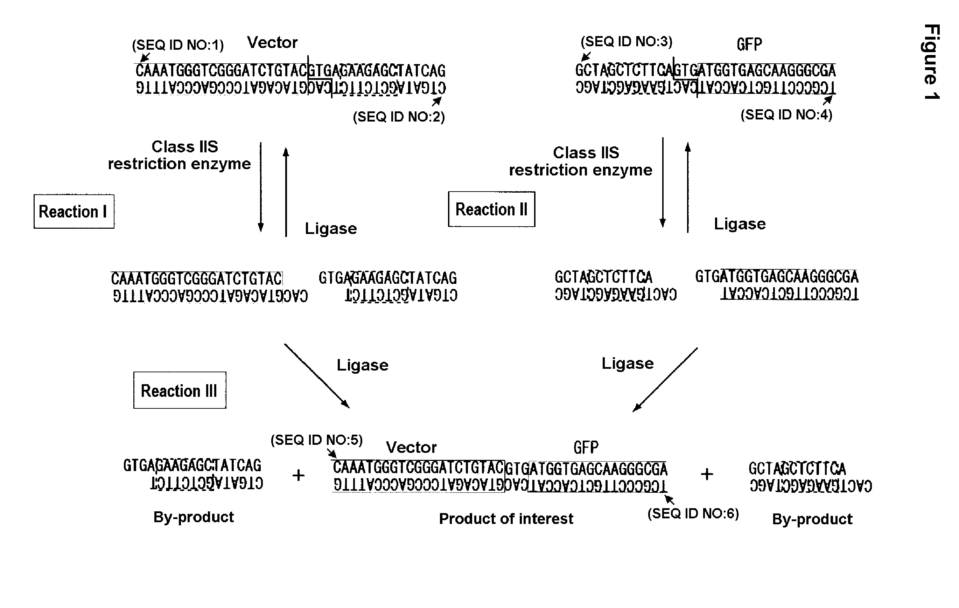

Figure 1 is a view illustrating each reaction step in a method for preparing a recombinant DNA according to the present invention. Here, the dashed rectangular frames indicate DNA recognition sites of Class-IIS restriction enzyme *Lgu*I, the step-like solid lines indicate DNA cleavage sites, and the frames surrounded by fine lines indicate sequences that specifically bind to template DNA.

First, when a DNA containing a DNA fragment of interest (in this figure, EGFP) is exposed to a restriction enzyme (in this figure, *Lgu*I that belongs to Class-IIS) in a reaction vessel (e.g., a test tube), both ends of the DNA containing the fragment are cleaved, giving the fragment having sticky ends (Reaction II). At the same time, a vector DNA (in this figure, a plasmid vector) is exposed to the same restriction enzyme in the same test tube, by which a linear vector having a site into which the DNA fragment of interest can be inserted (a site for inserting the fragment) is prepared (Reaction I).

If a DNA ligase (in this figure, a ligase) is also placed in the same test tube at the same time, cleavage by the restriction enzyme (Reactions I and II) and ligation by the DNA ligase (an opposing reaction to Reactions I and II) will progress simultaneously. In other words, an equilibrium reaction occurs where the cleavage and the ligation are repeated.

At this point, if the linear vector has sticky ends that are complementary to the sticky ends of the DNA fragment, the linear vector and the DNA fragment are ligated by the DNA ligase (Reaction III), and thus reaction for generating a circular vector DNA incorporating the fragment can also proceed. Since the recognition site of the Class-IIS restriction enzyme is no longer available in the circular recombinant vector DNA, the generated recombinant vector DNA does not undergo cleavage by the Class-IIS restriction enzyme. Thus, in Reaction III, only the ligation reaction by the DNA ligase progresses in an irreversible manner. Therefore, equilibrium between Reactions I and II shifts to the cleavage reaction side, and thus the equilibrium reaction does not occur anymore, and only one-way reaction towards the final product of interest, i.e., the recombinant DNA, takes place.

Accordingly, by using this method (FASTR method: Fully Automatic Single Tube Recombination) (former REMDI method: Restriction Enzyme = Mediated DNA Incorporation), cumbersome multiple steps for obtaining a DNA fragment of interest and a vector DNA can be omitted including, for example, a step for separately reacting each of the DNA fragment and the vector DNA with a restriction enzyme, a step for separating/purifying each reaction product by electrophoresis, a step for processing the DNA fragment including a dephosphorylation reaction and a step for mixing and ligating the both at an appropriately-adjusted molar ratio, and thus the recombinant DNA of interest can be produced efficiently.

This method also allows simultaneous insertion of multiple DNA fragments of interest into a vector DNA by placing multiple DNAs containing the fragments with different sequences. In order to allow the DNA fragments of interest to ligate to each other, sticky ends at both ends of each fragment should be complementary to sticky ends of another fragment. Furthermore, in order to ligate the DNA fragments of interest to the vector DNA, sticky ends of the fragments to be ligated into the vector should be complementary to the sticky ends at both ends of the linear vector DNA incorporating the fragments. Moreover, in order to bind the multiple DNA fragments of interest in a correct order, sticky ends of each fragment should be complementary only at correct ligation sites. As long as the DNA to be used has such intended sequences, all of the multiple DNA fragments of interest can simultaneously be introduced into the vector DNA to obtain a recombinant DNA through a single operation, which greatly saves the labor associated with the conventional procedure.

When the DNA containing the DNA fragment of interest and the vector DNA for inserting the fragment are amplified through PCR, the activity of a heat-resistant DNA polymerase remains in each PCR solution after the PCR, which may render the protruding ends of the amplified fragments to be blunted. Therefore, as a pre-treatment for the reactions (a) and (b), the amplified DNA fragments are preferably purified from each PCR solution in advance.

This method, however, requires a step of purifying the amplified products before inserting the DNA fragment of interest into the vector DNA fragment using the amplified DNA containing the fragment and the amplified vector DNA, which is inefficient. Hence, the present inventors have searched for a method which allows such purification method to be omitted, and found a method where the purification step can be omitted by using a DNA polymerase inhibitor or the like to speed up the DNA recombination.

Specifically, according to a preferred embodiment of the present invention, a DNA containing a DNA fragment of interest and a vector DNA for inserting the fragment are amplified through PCR, and then each PCR solution whose DNA polymerase activity has been inhibited by a polymerase inhibitor or the like can directly be subjected to reactions (a) and (b) described above, thereby carrying out DNA recombination more efficiently.

The term "inhibit" as used herein preferably means to completely and irreversibly eliminate the polymerase activity after PCR, but even if the activity is not completely eliminated, it is also used to mean, for example, temporarily suppressing a part of the DNA polymerase activity function for the purpose of preventing side reactions caused by the remaining DNA polymerase activity, such as blunting of the protruding ends of the amplified fragments after PCR.

Here, examples of chemical molecules of polymerase inhibitors that inhibit the heat-resistant DNA polymerase include aphidicolin, actinomycin D, mitomycin C, rifampicin 2, 3-dideoxythymidine 5-triphosphate (d2TTP), 1-β-D-arabinofuranosylcytosine 5-triphosphate (araCTP), mikanolide, L-homoserylaminoethanol, kaempferol 3-O-(6"-acetyl)-β-glucopyranoside (KAG), quercetin 3-O-(6"-acetyl)-β-glucopyranoside (QAG) and the like, although any compound may be used as long as it specifically inhibits the activity of the DNA polymerase.

In addition, an example of a DNA polymerase inhibitor that temporality neutralizes the activity of the DNA polymerase includes an anti-DNA polymerase antibody. Such an antibody is primarily used in Hot Start PCR method, but it is also utilizable in the present invention.

Examples of commercially available antibodies preferably used as a polymerase inhibitor in the present invention include, but not limited to, Taq DNA polymerase monoclonal antibody (AppliChem GmbH), anti-Taq monoclonal antibody (eEnzyme LLC), platinum Taq antibody (Invitrogen), Taq antibody (Novagen), JumpStart (registered trademark) Taq antibody (Sigma-Aldrich) and Taq polymerase monoclonal antibody (Takara). In addition, an antibody prepared using polymerase for PCR or a partial peptide thereof as an epitope may also be used as a DNA polymerase inhibitor.

Furthermore, a method for specifically breaking the polymerase by other physical method may also be employed. Since polymerases used for PCR generally have superior heat resistance, inactivating only with heat treatment is often insufficient. Due to stable property of DNA, however, prolonged heat treatment could specifically inhibit the polymerase activity. Change in pH or use of other neutralizing agent could also irreversibly inhibit only the polymerase activity.

Since the template DNA (usually plasmid DNA) is present in each of the solutions containing the PCR-amplified DNA containing a DNA fragment of interest and the vector DNA, if the solutions are directly used for DNA recombination, the occurrence rate of *E. coli* bearing the recombinant DNA of interest will be significantly reduced. However, since the plasmid DNA used as a template has been hemimethylated, only the template DNA can be digested, for example, with an enzyme such as *Dpn*I that causes cleavage reaction only when the nucleotide sequence to be recognized is methylated. Digestion of the template DNA remaining in the reaction solution with an enzyme such as *Dpn*I in the above-described manner allows efficient use of the DNA recombinant for transformation.

### EXAMPLES

Hereinafter, the present invention will be described in more detail by means of the following examples. These examples are merely provided for illustration and are not intended to limit the scope of the invention.

### Example 1: DNA recombination with LguI

### 1. Amplification of DNA fragment of interest and vector DNA fragment by PCR method

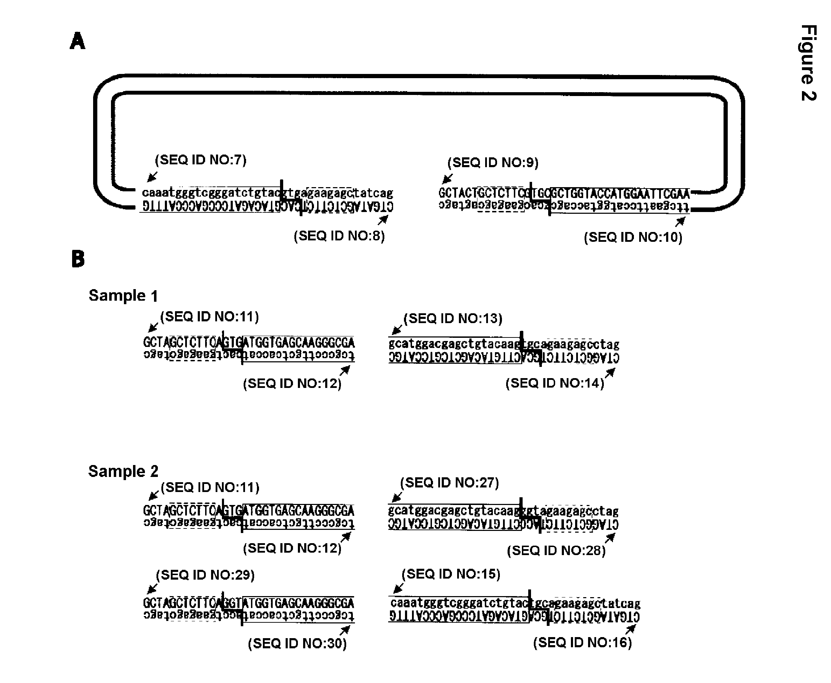

As shown in Figure 2, an EGFP sequence was used as a DNA fragment of interest and pRSET-B (Invitrogen) as a vector. To a reaction solution (120 mM Tris-HC1, 1 mM dNTP, 1.5 mM MgSO₄, 10 mM KCl, 6 mM (NH₄)₂SO₄ 0.1% TitonX-100, 0.1 mg/ml BSA), KOD plus DNA polymerase (Toyobo) was added. pcDNA3-EGFP and pRSET-B were used as templates for EGFP amplification and for pRSET-B amplification, respectively. The combinations of the primers are shown in Figure 2.

In Figure 2, the normal capital letters represent forward primer sequences while the upside down capital letters represent the reverse primer sequences. The lower-case letters conveniently represent the respective complementary sequences. The dashed rectangular frames indicate the DNA recognition sites of Class-IIS restriction enzyme *Lgu*I, the step-like solid lines indicate the DNA cleavage sites, and the frames surrounded by fine lines indicate sequences that specifically bind to the template DNAs. Moreover, A shows a primer set for pRSET-B (SEQ ID NOS:8 and 9) while B shows primer sets for EGFPs (Sample 1:SEQ ID NOS:11 and 14, Sample 2: SEQ ID NOS:11 and 28 and SEQ ID NOS:29 and 16).

The reaction cycles were as follows: a cycle of 94°C for 2 minutes; 35 cycles of 94°C for 15 seconds, 60°C for 30 seconds and 68°C for 90 seconds; and a cycle of 68°C for 5 minutes. One EGFP fragment was amplified for Sample 1, and two EGFP fragments having different sticky end sequences were amplified for Sample 2, respectively.

### 2. Separation/purification of reaction products by electrophoresis

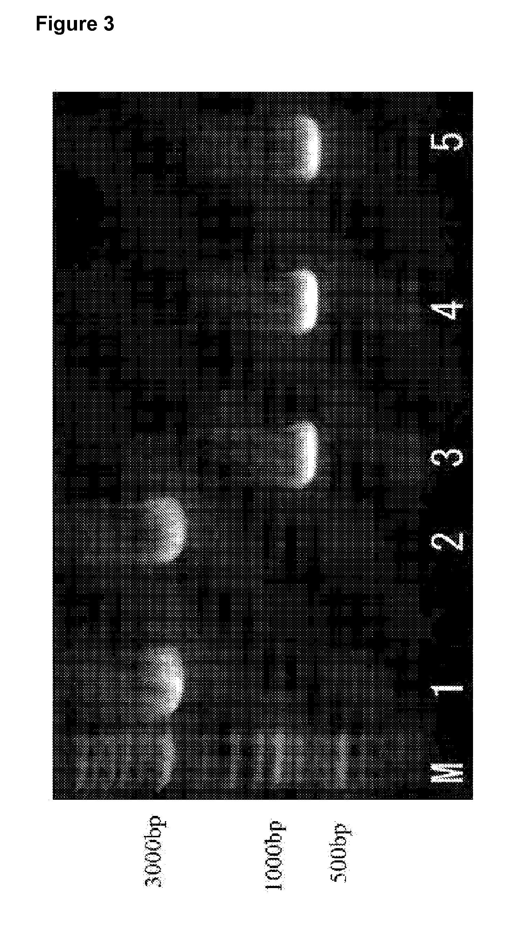

The PCR products were separated on an agarose gel (1% agarose, 1xTAE (40mM Tris-acetate (pH8.3), 1mM EDTA)) to purify each fragment (QIAquick, Invitrogen). The results of electrophoresis of the PCR products are shown in Figure 3. Here, Lanes 1 and 2 show the pRSET vector, while Lanes 3 to 5 show the EGFP fragments. In addition, M represents a DNA marker (2-log DNA marker, NEB).

### 3. Preparation of recombinant vector DNA

Each of the DNA fragments purified in the above step 2, a restriction enzyme and a DNA ligase were mixed together and left to stand at room temperature. Specifically, to a reaction solution (25 mM Tris-HCl, 16 mM Tris-acetate, 33 mM K-acetate, 5 mM MgCl₂, 5 mM Mg-acetate, 5 mM DTT, 50 µM 2-mercaptoethanol, 60 µg/ml BSA, 0.5 mM ATP), the DNA fragment, a DNA ligase (1 µl, T4 DNA Ligase, NEB) and a restriction enzyme (5 µl, *Lgu*I, Fermentas) were added and the resultant solution was left to stand for two hours.

### 4. Expression of recombinant vector DNA in E. coli

Five µl of the reaction solution containing the recombinant vector DNA prepared in 3. above was added to competent *E*. *coli* (XL 10 Gold, Stratagene), left to stand on ice for 10 minutes, followed by heat treatment at 42°C for 45 seconds. A 200 µl LB medium was added to the *E*. *coli,* cultured at 37°C for an hour, spread on a 1x LB plate (containing 100 µg/ml ampicillin), and cultured at 37°C for 15 hours.

Eight *E*. *coli* colonies were selected from each sample (Samples 1 and 2) for a total of 16 colonies. A part of each colony was suspended in a PCR solution (1x GoTaq Premix (Promega), T7 forward primer, and pRSET reverse primer) to perform colony direct PCR.

### 5. Identification of recombinant DNA

The PCR products were subjected to electrophoresis on an agarose gel to measure the fragment lengths of the PCR products.

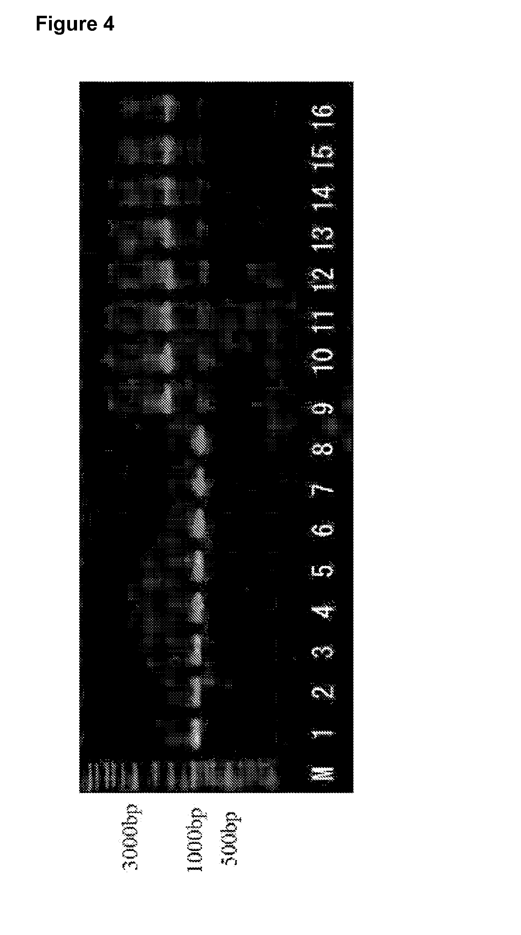

Figure 4 shows the results of the colony direct PCR. Lanes 1 to 8 show the fragments amplified from Sample 1-derived colonies, and Lanes 9 to 16 represent the fragments amplified from Sample 2-derived colonies. The 950 bp bands of Lanes 1 to 8 indicate that the Sample 1 fragments have been incorporated. For Lanes 9 to 16, since the two fragments of Sample 2 were linked together and simultaneously incorporated, bands of about twice (1650bp) the length of the single-fragment sample were detected. M represents the DNA size marker (2-log DNA marker, NEB). For each sample, 8 positive clones were obtained from 8 colonies.

### 6. Sequencing of clone sequences

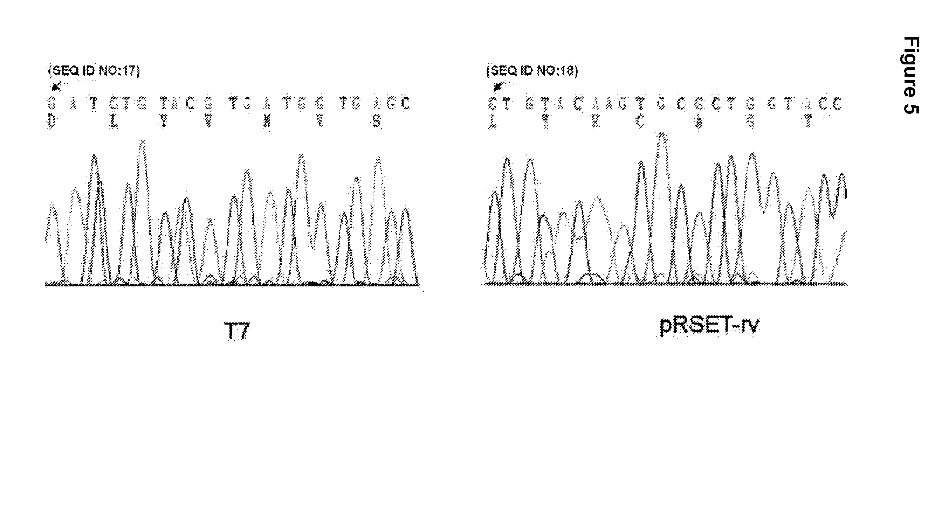

The results from sequencing the nucleotide sequences in the vicinity of the ligation site between the DNA fragment of interest and the vector using T7 forward and pRSET reverse primers are shown in Figure 5. The results from sequencing the nucleotide sequence assure that binding was formed, which supports the principles.

### Example 2: DNA recombination with BfuAI

### 1. Amplification of DNA fragment of interest and vector DNA fragment by PCR method

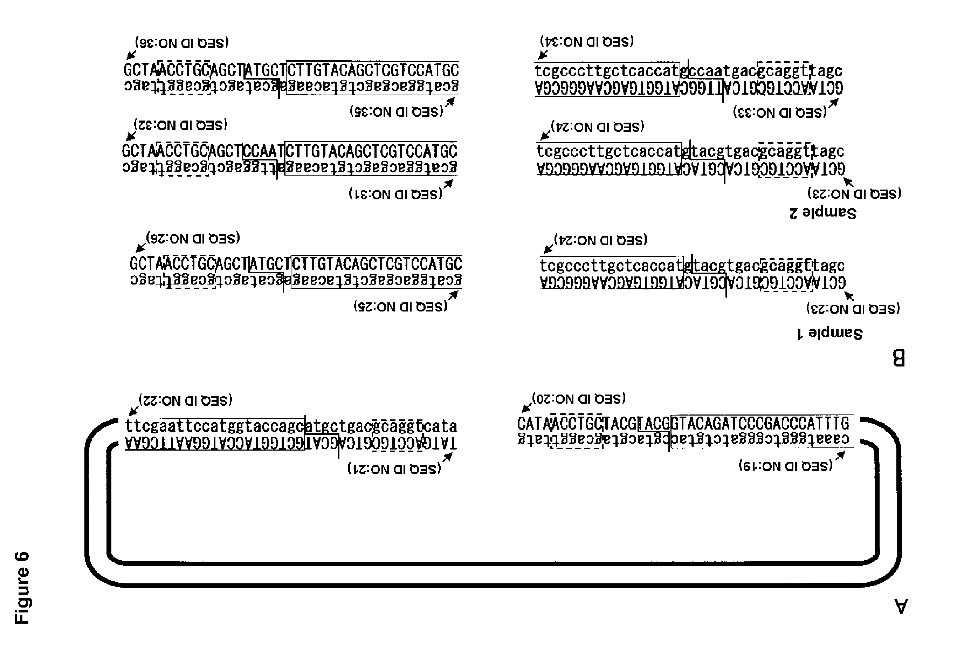

An EGFP sequence was used as a DNA fragment of interest and pRSET-B (Invitrogen) as a vector. To a reaction solution (120 mM Tris-HCl, 1 mM dNTP, 1.5 mM MgSO₄, 10 mM KCl, 6 mM (NH₄)₂SO₄, 0.1% TitonX-100, 0.1 mg/ml BSA), KOD plus DNA polymerase (Toyobo) was added. The reaction cycle was as follows: a cycle of 94°C for 2 minutes; 35 cycles of 94°C for 15 seconds, 60°C for 30 seconds and 68°C for 90 second; and a cycle of 68°C for 5 minutes. pcDNA3-EGFP and pRSET-B were used as templates for EGFP amplification and pRSET-B amplification, respectively. The combinations of the primers are shown in Figure 6.

In Figure 6, the normal capital letters represent forward primer sequences while the upside down capital letters represent the reverse primer sequences. The lower-case letters conveniently represent the respective complementary sequences. The dashed rectangular frames indicate the recognition sites of Class-IIS restriction enzyme *Bfu*AI, the step-like solid lines indicate the cleavage sites, and the frames surrounded by fine lines indicate sequences that specifically bind to template DNA. Moreover, A shows a primer set for pRSET-B (SEQ ID NOS:20 and 21) while B shows primer sets for EGFPs (Sample 1: SEQ ID NOS:23 and 26, Sample 2: SEQ ID NOS:23 and 32, and SEQ ID NOS:33 and 36). Two EGFP fragments having different sticky end sequences were amplified for Sample 2.

### 2. Separation/purification of reaction products by electrophoresis

The PCR products were separated on an agarose gel (1% agarose, 1xTAE) to purify each fragment (QIAquick, QIAGEN).

### 3. Preparation of recombinant vector DNA

Each of the DNA fragments purified in the above step 2, a restriction enzyme and a DNA ligase were mixed together and left to stand at room temperature. Specifically, to a reaction solution (25 mM Tris-HCl, 16 mM Tris-acetate, 33 mM K-acetate, 5 mM MgCl₂, 5 mM Mg-acetate, 5 mM DTT, 50 µM 2-mercaptoethanol, 60 µg/ml BSA, 0.5 mM ATP), the DNA fragment, a DNA ligase (1 µl, T4 DNA Ligase, NEB) and a restriction enzyme (5 µl, *Bfu*AI, NEB) were added and the resultant solution was left to stand for two hours. Subsequently, the reaction solution was heat-inactivated.

### 4. Expression of recombinant vector DNA in E. coli

Five µl of the reaction solution containing the recombinant vector DNA prepared in 3. above was added to competent *E*. *coli* (XL 10 Gold, Stratagene), left to stand on ice for 10 minutes, followed by heat treatment at 42°C for 45 seconds. A 200 µl LB medium was added to the *E*. *coli,* cultured at 37°C for an hour, spread on a 1x LB plate (containing 100 µg/ml ampicillin), and cultured at 37°C for 15 hours.

Eight *E*. *coli* colonies were selected from each sample (Samples 1 and 2) for a total of 16 colonies. A part of each colony was suspended in a PCR solution (1x GoTaq Premix (Promega), T7 forward primer, and pRSET reverse primer) to perform colony direct PCR.

### 5. Identification of recombinant DNA

The PCR products were subjected to electrophoresis on an agarose gel to measure the fragment lengths of the PCR products.

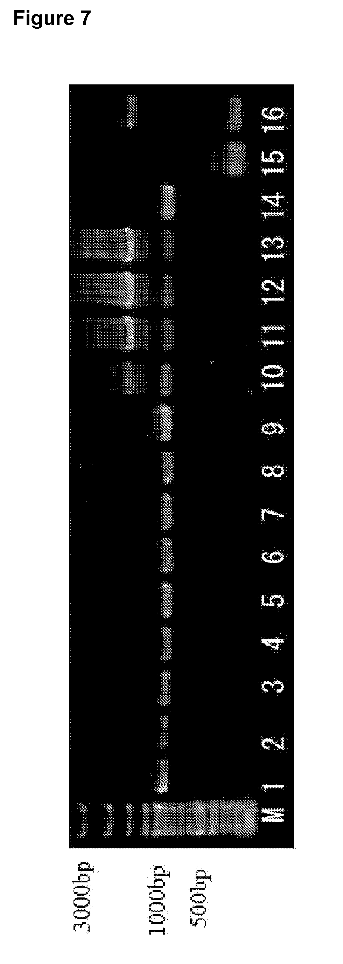

Figure 7 shows the results of the colony direct PCR. Lanes 1 to 8 show the fragments amplified from Sample 1-derived colonies, and Lanes 9 to 16 represent the fragments amplified from Sample 2-derived colonies. The bands near 950 bp indicate that the Sample 1 fragments (EGFP) have been incorporated. The bands near 1650 bp have about twice the length of the single-fragment sample (950 bp), which indicates that the two fragments (EGFP x 2) of Sample 2 were linked together and simultaneously incorporated. M represents the DNA size marker (2-log DNA marker, NEB). Eight and five positive clones were obtained out of 8 colonies each of Samples 1 and 2, respectively.

### Example 3: Speeding-up of DNA recombination with polymerase inhibitor

### 1. Amplification of DNA fragment of interest and vector DNA fragment by PCR method

In the same manner as Example 1, a DNA fragment of a gene of interest and a DNA fragment of a vector were amplified using PCR method. An EGFP sequence was used as the gene of interest and pRSET-B (*Lgu*I-site-defective, Invitrogen) as the vector. To a reaction solution (120 mM Tris-HCl, 1 mM dNTP, 1.5 mM MgSO₄, 10 mM KCl, 6 mM (NH₄)₂SO₄ 0.1%TitonX-100, 0.1 mg/ml BSA), KOD plus DNA polymerase (Toyobo) was added. The combinations of the primers were as indicated in Figure 2. The reaction cycles were as follows: a cycle of 94°C for 2 minutes; 45 cycles of 94°C for 15 seconds, 60°C for 30 seconds and 68°C for 90 seconds; and a cycle of 68°C for 5 minutes. pcDNA3-EGFP and pRSET-B were used as a template for EGFP amplification and pRSET-B amplification, respectively.

### 2. Preparation of recombinant vector DNA

To a reaction solution (25 mM Tris-HCl, 16 mM Tris-acetate, 33 mM K-acetate, 5 mM MgCl₂, 5 mM Mg-acetate, 5 mM DTT, 50 µM 2-mercaptoethanol, 60 µg/ml BSA, 0.5 mM ATP), solutions were added respectively containing the DNA fragment of interest and the vector DNA fragment that were separately amplified in 1. above, and further a DNA ligase (1 µl, T4 DNA Ligase, NEB), a restriction enzyme (5 µl, *Lgu*I, Fermentas) and a polymerase inhibitor (5 mM, aphidicolin, Cosmo Bio Co., Ltd.) were added thereto and the resultant solution was left to stand at room temperature for 2 hours.

One µl of *Dpn*I (NEB) was further added to the above-mentioned reaction solution, and the resultant solution was left to stand at 37°C for 30 minutes to digest only the template DNA.

### 3. Expression of recombinant vector DNA in E. coli

A 2.5 µl reaction solution containing the recombinant vector DNA prepared in the above step 2 was transformed into 25 µl of JM109 competent *E*. *coli* (Stratagene), and spread on a LB plate.

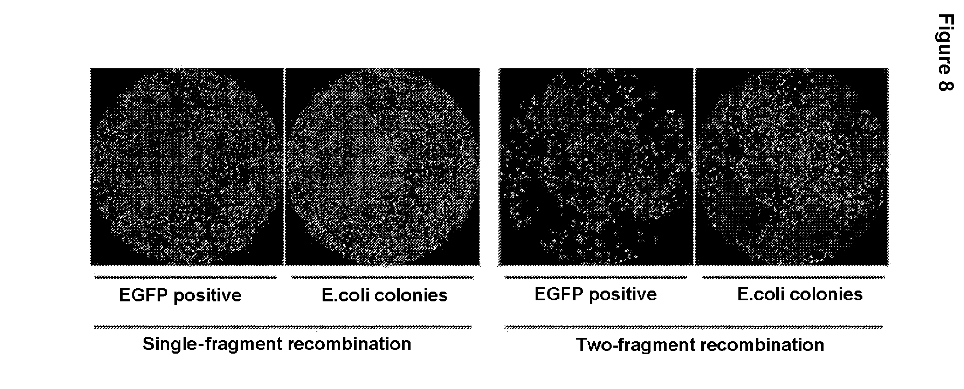

On the following day, the LB plate was subjected to fluorescence photography using an image analyzer (LAS-1000, Fuji Film) to count the number of whole colonies and the number of fluorescent colonies using an image analysis software (MetaMorph, Molecular Devices). In Figure 8, the whole colonies and the fluorescent colonies are shown as *E*. *coli* colonies and EGFP positive colonies, respectively.

EGFP positive colonies were 2769 out of 3735 colonies for the single-fragment transformation (Figure 2: Sample 1), and 688 out of 936 colonies for the two-fragment transformation (Figure 2: Sample 2), with transformation efficiency being 74.1% and 73.5%, respectively.

According to the above experiments, inhibition of the remaining polymerase activity with an agent or the like was found to allow PCR products to be used directly for a transformation reaction.

### INDUSTRIAL APPLICABILITY

The present invention provides a simplified and efficiently improved DNA recombination method. Use of such a DNA recombination method of the present invention allows efficient productions of various enzymes, cytokines such as interferon and interleukin, and useful proteins such as hormones.

### SEQUENCE LISTING FREE TEXT

### SEQ ID NOS:1 to 36: Synthetic DNA

## Claims

1. A method for preparing a recombinant DNA by inserting a DNA fragment of interest into a vector DNA, comprising the step of carrying out the following reactions at the same reacting location at the substantially simultaneous time:
(a) a reaction for simultaneously cleaving a site of the vector DNA for inserting the fragment and a DNA containing the fragment in the presence of a restriction enzyme whose DNA recognition site and DNA cleavage site are discrete; and
(b) a reaction for inserting the fragment into the vector DNA in the presence of a DNA ligase.

2. A method for preparing a recombinant DNA by inserting a DNA fragment of interest into a vector DNA, comprising the step of carrying out the following reactions at the same reacting location at the substantially simultaneous time:
(a) a reaction for simultaneously cleaving a site of the vector DNA for inserting the fragment and both ends of a DNA containing the fragment in the presence of a restriction enzyme whose DNA recognition site and DNA cleavage site are discrete; and
(b) a reaction for inserting the fragment into the vector DNA in the presence of a DNA ligase.

3. The method according to Claim 1 or 2, wherein the restriction enzyme recognizes and cleaves, as the DNA cleavage site, a site at a certain distance from the DNA recognition site.

4. The method according to Claim 1 or 2, wherein the restriction enzyme is a Class-IIS restriction enzyme.

5. The method according to any one of Claims 1 to 4, wherein the DNA fragment of interest consists of multiple DNA fragments each having a different sequence, and wherein the multiple DNA fragments are simultaneously inserted into the vector DNA.

6. The method according to any one of Claims 1 to 5, wherein the reactions (a) and (b) are carried out by using a solution obtained by adding a DNA polymerase inhibitor to a PCR solution containing a PCR-amplified DNA including the DNA fragment of interest and a PCR-amplified vector DNA.


SEQUENCE LISTING

<110>   National University Corporation Hokkaido University

<120>   Method for Preparing Recombinant DNA

<130>   G10-0010

<140>   PCT/JP2008/064482
<140>   2008-08-12

<150>   JP 2007-215238
<151>   2007-08-21

<160>   36

<170>   PatentIn version 3.4

<210>  1

<211>  38

<212>  DNA

<213>  Artificial

<220>

<223>  synthetic DNA

<400>  1

caaatgggtc gggatctgta cgtgagaaga gctatcag                                38

<210>  2

<211>  38

<212>  DNA

<213>  Artificial

<220>

<223>  synthetic DNA

<400>  2

ctgatagctc ttctcacgta cagatcccga cccatttg                                38

<210>  3

<211>  32

<212>  DNA

<213>  Artificial

<220>

<223>  synthetic DNA

<400>  3

gctagctctt cagtgatggt gagcaagggc ga                                      32

<210>  4

<211>  32

<212>  DNA

<213>  Artificial

<220>

<223>  synthetic DNA

<400>  4

tcgcccttgc tcaccatcac tgaagagcta gc                                    32

<210>  5

<211>  41

<212>  DNA

<213>  Artificial

<220>

<223>  synthetic DNA

<400>  5

caaatgggtc gggatctgta cgtgatggtg agcaagggcg a                41

<210>  6

<211>  41

<212>  DNA

<213>  Artificial

<220>

<223>  synthetic DNA

<400>  6

tcgcccttgc tcaccatcac gtacagatcc cgacccattt g                41

<210>  7

<211>  38

<212>  DNA

<213>  Artificial

<220>

<223>  synthetic DNA

<400>  7

caaatgggtc gggatctgta cgtgagaaga gctatcag                                38

<210>  8

<211>  38

<212>  DNA

<213>  Artificial

<220>

<223>   synthetic DNA

<400>   8

ctgatagctc ttctcacgta cagatcccga cccatttg                                38

<210>   9

<211>   38

<212>   DNA

<213>   Artificial

<220>

<223>   synthetic DNA

<400>   9

gctactgctc ttcgtgcgct ggtaccatgg aattcgaa                                38

<210>  10

<211>  38

<212>  DNA

<213>  Artificial

<220>

<223>  synthetic DNA

<400>  10

ttcgaattcc atggtaccag cgcacgaaga gcagtagc                                38

<210>  11

<211>  32

<212>  DNA

<213>  Artificial

<220>

<223>  synthetic DNA

<400>  11

gctagctctt cagtgatggt gagcaagggc ga                                    32

<210>  12

<211>  32

<212>  DNA

<213>  Artificial

<220>

<223>  synthetic DNA

<400>  12

tcgcccttgc tcaccatcac tgaagagcta gc                                    32

<210> 13

<211> 35

<212> DNA

<213> Artificial

<220>

<223> synthetic DNA

<400> 13

gcatggacga gctgtacaag tgcagaagag cctag 35

<210> 14

<211> 35

<212> DNA

<213> Artificial

<220>

<223>   synthetic DNA

<400>   14

ctaggctctt ctgcacttgt acagctcgtc catgc                                    35

<210>   15

<211>   38

<212>   DNA

<213>   Artificial

<220>

<223>   synthetic DNA

<400>   15

caaatgggtc gggatctgta ctgcagaaga gctatcag                                 38

<210> 16

<211> 38

<212> DNA

<213> Artificial

<220>

<223> synthetic DNA

<400> 16

ctgatagctc ttctgcagta cagatcccga cccatttg 38

<210> 17

<211> 21

<212> DNA

<213> Artificial

<220>

<223>  synthetic DNA

<400>  17

gatctgtacg tgatggtgag c                                                21

<210>  18

<211>  21

<212>  DNA

<213>  Artificial

<220>

<223>  synthetic DNA

<400>  18

ctgtacaagt gcgctggtac c                                                21

<210>  19

<211>  39

<212>  DNA

<213>  Artificial

<220>

<223>  synthetic DNA

<400>  19

caaatgggtc gggatctgta ccgtacgtag caggttatg                                    39

<210>  20

<211>  39

<212>  DNA

<213>  Artificial

<220>

<223>	synthetic DNA

<400>	20

cataacctgc tacgtacggt acagatcccg acccatttg	39

<210>	21

<211>	39

<212>	DNA

<213>	Artificial

<220>

<223>	synthetic DNA

<400>	21

tatgacctgc gtcagcatgc tggtaccatg gaattcgaa	39

<210> 22

<211> 39

<212> DNA

<213> Artificial

<220>

<223> synthetic DNA

<400> 22

ttcgaattcc atggtaccag catgctgacg caggtcata 39

<210> 23

<211> 36

<212> DNA

<213> Artificial

<220>

<223>  synthetic DNA

<400>  23

gctaacctgc gtcacgtaca tggtgagcaa gggcga                                36

<210>  24

<211>  36

<212>  DNA

<213>  Artificial

<220>

<223>  synthetic DNA

<400>  24

tcgcccttgc tcaccatgta cgtgacgcag gttagc                                36

<210>  25

<211>  39

<212>  DNA

<213>  Artificial

<220>

<223>  synthetic DNA

<400>  25

gcatggacga gctgtacaag agcatagctg caggttagc                                    39

<210>  26

<211>  39

<212>  DNA

<213>  Artificial

<220>

<223>  synthetic DNA

<400>  26

gctaacctgc agctatgctc ttgtacagct cgtccatgc                                    39

<210>  27

<211>  35

<212>  DNA

<213>  Artificial

<220>

<223>  synthetic DNA

<400>  27

gcatggacga gctgtacaag ggtagaagag cctag                                        35

<210> 28

<211> 35

<212> DNA

<213> Artificial

<220>

<223> synthetic DNA

<400> 28

ctaggctctt ctacccttgt acagctcgtc catgc 35

<210> 29

<211> 32

<212> DNA

<213> Artificial

<220>

<223>   synthetic DNA

<400>   29

gctagctctt caggtatggt gagcaagggc ga                                    32

<210>   30

<211>   32

<212>   DNA

<213>   Artificial

<220>

<223>   synthetic DNA

<400>   30

tcgcccttgc tcaccatacc tgaagagcta gc                                    32

<210> 31

<211> 39

<212> DNA

<213> Artificial

<220>

<223> synthetic DNA

<400> 31

gcatggacga gctgtacaag attggagctg caggttagc 39

<210> 32

<211> 39

<212> DNA

<213> Artificial

<220>

<223>   synthetic DNA

<400>   32

gctaacctgc agctccaatc ttgtacagct cgtccatgc                                        39

<210>   33

<211>   36

<212>   DNA

<213>   Artificial

<220>

<223>   synthetic DNA

<400>   33

gctaacctgc gtcattggca tggtgagcaa gggcga                                           36

<210>  34

<211>  36

<212>  DNA

<213>  Artificial

<220>

<223>  synthetic DNA

<400>  34

tcgcccttgc tcaccatgcc aatgacgcag gttagc                                36

<210>  35

<211>  39

<212>  DNA

<213>  Artificial

<220>

<223>   synthetic DNA

<400>   35

gcatggacga gctgtacaag agcatagctg caggttagc                                    39

<210>   36

<211>   39

<212>   DNA

<213>   Artificial

<220>

<223>   synthetic DNA

<400>   36

gctaacctgc agctatgctc ttgtacagct cgtccatgc                                    39